(19) **Europäisches Patentamt / European Patent Office / Office européen des brevets**

(11) **EP 4 324 391 B1**

(12) **EUROPEAN PATENT SPECIFICATION**

(45) Date of publication and mention of the grant of the patent:
**16.10.2024 Bulletin 2024/42**

(21) Application number: **23188323.2**

(22) Date of filing: **28.07.2023**

(51) International Patent Classification (IPC):
**A61B 5/11** (2006.01)     **A61B 5/00** (2006.01)

(52) Cooperative Patent Classification (CPC):
**A61B 5/112; A61B 5/6823; A61B 5/743;**
A61B 2562/0219

(54) **GAIT EVALUATION SYSTEM AND GAIT EVALUATION METHOD**

GANGBEURTEILUNGSSYSTEM UND GANGBEURTEILUNGSVERFAHREN

SYSTÈME D'ÉVALUATION DE DÉMARCHE ET PROCÉDÉ D'ÉVALUATION DE DÉMARCHE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.08.2022 JP 2022129190**

(43) Date of publication of application:
**21.02.2024 Bulletin 2024/08**

(73) Proprietor: **ASICS Corporation**
**Kobe-shi, Hyogo 650-8555 (JP)**

(72) Inventors:
• **KUSANO, Ken**
  **Kobe-shi, 6508555 (JP)**
• **ICHIKAWA, Masaru**
  **Kobe-shi, 6508555 (JP)**

• **TAGAWA, Takehiro**
  **Kobe-shi, 6508555 (JP)**

(74) Representative: **TBK**
**Bavariaring 4-6**
**80336 München (DE)**

(56) References cited:
AU-A4- 2021 107 366     JP-A- 2015 202 140
US-A1- 2017 202 485

• **KULKARNI SNEHAL ET AL: "Basic gait pattern and impact of fall risk factors on gait among older adults in India", GAIT & POSTURE, ELSEVIER, AMSTERDAM, NL, vol. 88, 30 April 2021 (2021-04-30), pages 16 - 21, XP086698430, ISSN: 0966-6362, [retrieved on 20210430], DOI: 10.1016/J.GAITPOST.2021.04.043**

## Description

BACKGROUND

1. Field of the Invention

The present disclosure relates to a gait evaluation system. In particular, the present disclosure relates to a system for analyzing the effects of walking exercise.

2. Description of the Related Art

With the increasing health consciousness of people, walking is gaining popularity. Especially in recent years, with widespread use of smartphones and watches with built-in Global Positioning System (GPS) modules, anyone can easily record walking exercise logs (hereinafter, also referred to as "walking logs"). The use of such walking logs motivates people to continue walking and make it a habit, which is boosting the popularity of walking.

There is a known technology of capturing images of a user's strength training motions with an image capture device and calculating joint positions and angles from the captured images to estimate the training effects (see Japanese Unexamined Patent Application Publication No. 2022-051173 A, for example). There is also a known technology of calculating, based on detection results of a stretch sensor that detects stretching and contraction of an attachment part attached to a body part, such as a knee joint, the kinetic energy at the attachment part (see Japanese Unexamined Patent Application Publication No. 2020-174946 A, for example).

Furthermore, AU 2021 107 366 A4 discloses a system and method for determining quantitative gait scores from combinations of gait metrics, termed 'Combined Mobility Score'. Thereby, detected signals are analyzed to determine said 'Combined Mobility Score' for the subject from a combination of metrics in gait categories i-iv, namely: i. daily step count; ii. one or more of gait velocity, step length, stride length, and cadence; iii. one or more of step length asymmetry, step time asymmetry, single support time variability; and iv. walking orientation randomness metric.

Information recorded as a walking log is often information related mainly to the amount of walking, such as walking time and walking distance, and is not necessarily information related to the quality of walking exercise. In this regard, even if the technology of Japanese Unexamined Patent Application Publication No. 2022-051173 A is used to evaluate the quality of walking exercise, a special environment in which images of the user's exercise can be captured is required, so that the image capturing cannot be easily performed outdoors by the user himself or herself. Also, even if the technology of Japanese Unexamined Patent Application Publication No. 2020-174946 A is used to calculate the kinetic energy, since a dedicated device must be attached to a region that stretches and contracts, it cannot be said that the quality of walking exercise can be easily measured.

SUMMARY

The present disclosure has been made in view of such a situation, and a purpose thereof is to provide a technology for measuring the quantity and quality of walking exercise easily and estimating the exercise effects.

To solve the problem above, a gait evaluation system according to one aspect of the present disclosure includes: a quantitative measurement value acquirer that acquires a measurement value of a quantitative parameter indicating an amount of walking, measured by a measurement device worn by a subject; a qualitative measurement value acquirer that acquires at least measurement values of a stride length, of an arm swing angle and a cadence as qualitative parameters indicating a walking form, measured by a measurement device worn by the subject; a quantitative result determination unit that determines an evaluation result regarding an amount of walking, based on a measurement value of a quantitative parameter; a qualitative result determination unit that determines an evaluation result regarding a walking form, based on a predetermined evaluation model that reflects the degree of approximation to a form tendency with which a walking exercise effect is likely to be achieved, and a measurement value of a qualitative parameter; and an output unit that outputs walking result information including the evaluation result regarding the amount of walking and the evaluation result regarding the walking form, wherein the qualitative result determination unit (96) determines the evaluation result regarding the walking form using, as the predetermined evaluation model, an evaluation formula for obtaining the sum of a value obtained by adding a weight to the ratio of the subject's stride-height ratio to an average stride-height ratio of a corresponding attributed level, and a value of the ratio of the subject's cadence to an average cadence of the corresponding attributed level, and an evaluation formula set such that a weight is added at least to a measurement value of the arm swing angle.

Another aspect of the present disclosure also relates to a gait evaluation system. The gait evaluation system includes: a quantitative measurement value acquirer that acquires a measurement value of a quantitative parameter indicating an amount of walking, measured by a measurement device worn by a subject; a qualitative measurement value acquirer that acquires at least measurement values of pelvic movement, of an arm swing angle and a cadence as qualitative parameters indicating a walking form, measured by a measurement device worn by the subject; a quantitative result determination unit that determines an evaluation result regarding an amount of walking, based on a measurement value of a quantitative parameter; a qualitative result determination unit that determines an evaluation result regarding a walking form, based on a predetermined evaluation model that reflects the degree of approximation to a form tendency with which a walking exercise effect is likely to be achieved, and a measurement value of a qualitative parameter; and an output unit that outputs walking result information including the evaluation result regarding the amount of walking and the evaluation result regarding the walking form, wherein the qualitative result determination unit (96) determines the evaluation result regarding the walking form using, as the predetermined evaluation model, an evaluation formula for obtaining the sum of a value obtained by adding a weight to the ratio of the subject's stride-height ratio to an average stride-height ratio of a corresponding attributed level, and a value of the ratio of the subject's cadence to an average cadence of the corresponding attributed level, and an evaluation formula set such that a weight is added at least to a measurement value of the arm swing angle.

Yet another aspect of the present disclosure relates to a gait evaluation method. The method includes: acquiring a measurement value of a quantitative parameter indicating an amount of walking, measured by a measurement device worn by a subject; acquiring at least measurement values of a stride length, of an arm swing angle and a cadence as qualitative parameters indicating a walking form, measured by a measurement device worn by the subject; determining an evaluation result regarding an amount of walking, based on a measurement value of a quantitative parameter; determining an evaluation result regarding a walking form, based on a predetermined evaluation model that reflects the degree of approximation to a form tendency with which a walking exercise effect is likely to be achieved, and a measurement value of a qualitative parameter; and outputting walking result information including the evaluation result regarding the amount of walking and the evaluation result regarding the walking form, wherein the qualitative result determination unit (96) determines the evaluation result regarding the walking form using, as the predetermined evaluation model, an evaluation formula for obtaining the sum of a value obtained by adding a weight to the ratio of the subject's stride-height ratio to an average stride-height ratio of a corresponding attributed level, and a value of the ratio of the subject's cadence to an average cadence of the corresponding attributed level, and an evaluation formula set such that a weight is added at least to a measurement value of the arm swing angle.

BRIEF DESCRIPTION OF THE DRAWINGS

Embodiments will now be described, by way of example only, with reference to the accompanying drawings which are meant to be exemplary, not limiting, and wherein like elements are numbered alike in several FIGURES, in which:

FIG. 1 illustrates a configuration of a gait evaluation system;

FIG. 2 is a functional block diagram that shows each configuration in the gait evaluation system;

FIG. 3 shows relationships between the walking speed, stride length, and muscle activity level;

FIG. 4 illustrates a screen example for displaying a muscle activity level estimate;

FIG. 5 illustrates a screen example for displaying measurement values as qualitative parameters;

FIG. 6 illustrates a screen example for displaying exercise effects divided based on a quantitative parameter and a qualitative parameter; and

FIG. 7 illustrates a screen example for displaying an exercise effect on each body part.

DETAILED DESCRIPTION OF THE INVENTION

The disclosure will now be described by reference to the preferred embodiments. This does not intend to limit the scope of the present disclosure, but to exemplify the disclosure.

In the following, the present disclosure will be described based on a preferred embodiment with reference to each drawing. In the embodiment and modifications, like reference characters denote like or corresponding constituting elements, and the repetitive description will be omitted as appropriate.

First Embodiment

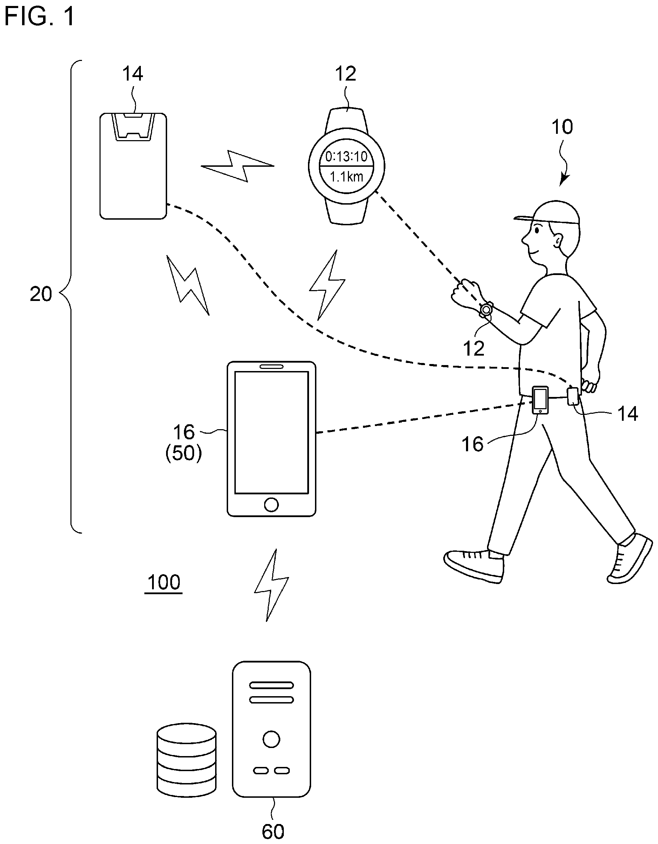

FIG. 1 illustrates a configuration of a gait evaluation system 100. The gait evaluation system 100 includes a wristwatch-type device 12, a waist-mounted device 14, and an information terminal-type device 16, which can be worn by a subject 10 who performs walking exercise, and an information management server 60. The wristwatch-type device 12, waist-mounted device 14, and information terminal-type device 16 are also collectively referred to as measurement devices 20. The wristwatch-type device 12 is a sports watch or a smart watch that can measure position information, motion information, and the like. The waist-mounted device 14 is a motion sensor that can be attached to the vicinity of the waist of the subject 10 to measure position information and motion information. The information terminal-type device 16 is a portable information terminal, such as a smartphone, that can measure position information and motion information while held in a pocket or the like by the subject 10. A measurement device 20 is not limited to a device such as the wristwatch-type device 12, waist-mounted device 14, or information terminal-type device 16 and may also be a belt-type device that can be worn around the chest, a wrist, the waist, or an arm of the subject to acquire position information and motion information. Further, various other wearable devices may be used as the measurement devices 20.

The subject 10 performs walking exercise while wearing at least one or all of the wristwatch-type device 12, waist-mounted device 14, and information terminal-type device 16 as the measurement devices 20. Each measurement device 20 synchronizes information via communication with the information management server 60. However, the communication means for the wristwatch-type device 12 and the waist-mounted device 14 among the measurement devices 20 is short-range wireless communication; therefore, instead of communicating directly with the information management server 60, the devices synchronize information with the information terminal-type device 16 (which also functions as an "information terminal 50" detailed later), and the information terminal 50 then synchronizes information with the information management server 60. Thus, since the wristwatch-type device 12 and the waist-mounted device 14 transmit information to the information management server 60 via synchronization to the information terminal 50, it is premised that the information terminal 50 is possessed. However, the information terminal 50 need not necessarily be worn during walking exercise, and it is sufficient if the synchronization with the information terminal 50 can be performed after the exercise. As a modification, after the waist-mounted device 14 synchronizes information with the wristwatch-type device 12 via near-field wireless communication, the wristwatch-type device 12 may further synchronize the information with the information terminal-type device 16 (information terminal 50) via near-field wireless communication.

The subject 10 performs walking exercise while wearing a measurement device 20 and starts measurement and walking log recording by operating a button or the like of the measurement device 20 at the start of the walking exercise. During the walking exercise, the measurement device 20 measures, as walking time, the elapsed time from the start of recording with a timer and also records position information for each date and time at predetermined time intervals. The measurement device 20 also measures the number of steps per unit time with a built-in motion sensor. With the built-in motion sensor, the measurement device 20 further measures values of the arm swing angle, pelvic movement, left and right walking tempos, and the like.

When the subject 10 wears the wristwatch-type device 12 as a measurement device 20, values of the time information, position information, number of steps per unit time, arm swing angle, and the like are measured, and the walking log is recorded. When the subject 10 wears the waist-mounted device 14 as a measurement device 20, values of the time information, position information, number of steps per unit time, pelvic movement (such as the hip rotation angle, lateral hip tilt, and front-back hip bend), left and right walking tempos, and the like are measured, and the walking log is recorded. When the subject 10 wears the information terminal-type device 16 as a measurement device 20, values of the time information, position information, number of steps per unit time, and the like are measured, and the walking log is recorded.

After the completion of the walking exercise and the walking log recording, the measurement device 20 transmits, to the information management server 60, information such as the walking time, position information, number of steps, arm swing angle, pelvic movement, and left and right walking tempos, as the walking log. Also, the measurement device 20 may calculate information such as the walking time, walking distance, and walking speed, based on the time information and position information, and may transmit the walking log including such calculated information to the information management server 60.

The information management server 60 is a server computer that is connected to the Internet and transmits and receives data to and from the information terminals 50 of multiple subjects 10. The information management server 60 acquires, as walking log data of a subject 10 received from the corresponding information terminal 50, measurement values of the time information, position information, number of steps per unit time, arm swing angle, and the like, together with identification information and attribute information of the subject 10, and accumulates the walking log data together with various data and evaluation values calculated from the measurement values. In response to a request from an information terminal 50, the information management server 60 transmits the accumulated walking log data, evaluation values, and the like to the information terminal 50.

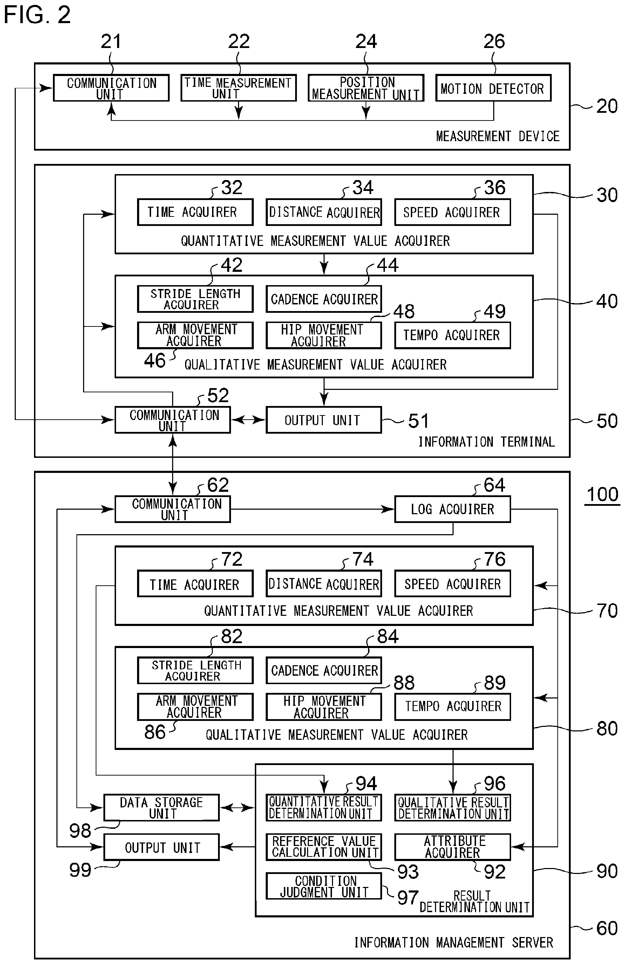

FIG. 2 is a functional block diagram that shows each configuration in the gait evaluation system 100. The gait evaluation system 100 in the present embodiment is constituted by a measurement device 20, an information terminal 50, and the information management server 60. The gait evaluation system 100 may be implemented by various hardware configurations and software configurations. For example, the gait evaluation system 100 may be constituted only by the information terminal 50, may be constituted by a combination of the information terminal 50 and the measurement device 20, or may be constituted by a combination of the information terminal 50 and the information management server 60. Also, the gait evaluation system 100 may be constituted by a combination of the information terminal 50, the measurement device 20, and the information management server 60, may be constituted by a combination of the measurement device 20 and the information management server 60, or may be constituted only by the information management server 60.

For example, on the premise that the walking exercise is measured and recorded as the walking log using a variety of general-purpose devices as the measurement device 20, the gait evaluation system 100 may be constituted only by the information terminal 50 and the information management server 60 or may be implemented as a stand-alone device that includes all software configurations included in the information terminal 50 and the information management server 60 shown in FIG. 2. Therefore, it is sufficient if the gait evaluation system 100 includes, regardless of its hardware configuration, at least the software configurations of the information terminal 50 and the information management server 60 shown in FIG. 2.

With regard to each of the measurement device 20, information terminal 50, and information management server 60, FIG. 2 shows functional blocks implemented by coordination of various hardware configurations and software configurations. Therefore, it will be understood by those skilled in the art that these functional blocks may be implemented in a variety of forms by hardware only, software only, or a combination thereof. The measurement device 20 is constituted by a combination of hardware, such as a microprocessor, memory, communication module, positioning module, and motion sensor. The information terminal 50 is constituted by a combination of hardware, such as a microprocessor, memory, display, communication module, positioning module, and motion sensor. The information management server 60 is constituted by a combination of hardware, such as a microprocessor, memory, display, and communication module. In the following, the functions of each of the measurement device 20, information terminal 50, and information management server 60 will be described.

The measurement device 20 is, for example, the waist-mounted device 14. The measurement device 20 includes a communication unit 21, a time measurement unit 22, a position measurement unit 24, and a motion detector 26. The time measurement unit 22 measures the walking start time, i.e., the walking time from the measurement start time, with counting by a timer. The position measurement unit 24 measures the current position based on position information received by a Global Positioning System (GPS) module from a satellite positioning system. The motion detector 26 detects, with a motion sensor, the number of steps, pelvic movement, and the like in the walking exercise of the subject 10. The measurement values of the number of steps include, besides the number of steps per unit time, the left and right walking tempos. The measurement values of the pelvic movement include the pelvic or hip rotation angle, lateral tilt angle of the pelvis or hips, and front-back bend angle of the pelvis or hips.

As the measurement device 20, the wristwatch-type device 12 or the information terminal-type device 16 may also be used. When the wristwatch-type device 12 is used as the measurement device 20, the motion detector 26 of the wristwatch-type device 12 detects, with a motion sensor, the number of steps, arm swing angle, and the like in the walking exercise of the subject 10. When the information terminal-type device 16 is used as the measurement device 20, the motion detector 26 of the information terminal-type device 16 detects, with a motion sensor, the number of steps in the walking exercise of the subject 10. The information terminal 50 may serve dually as the information terminal-type device 16 as the measurement device 20, and, in that case, a single mobile terminal such as a smartphone may have all the functions of both the measurement device 20 and the information terminal 50, for example. When the information terminal 50 is not used as the measurement device 20, the information terminal 50 may be not limited to a smartphone and may also be a tablet terminal or a personal computer owned by the subject 10.

The information terminal 50 includes a quantitative measurement value acquirer 30, a qualitative measurement value acquirer 40, an output unit 51, and a communication unit 52. The quantitative measurement value acquirer 30 receives, via the communication unit 52, information measured by the measurement device 20 worn by the subject 10. Based on the information received from the measurement device 20, the quantitative measurement value acquirer 30 acquires a measurement value of a quantitative parameter indicating the amount of walking. The quantitative measurement value acquirer 30 includes a time acquirer 32, a distance acquirer 34, and a speed acquirer 36.

The time acquirer 32 acquires, as a quantitative parameter indicating the amount of walking, the walking time of the subject 10 based on the information received from the time measurement unit 22. The distance acquirer 34 acquires, as a quantitative parameter indicating the amount of walking, the walking distance of the subject 10 based on the information received from the position measurement unit 24. The speed acquirer 36 acquires, as a quantitative parameter indicating the amount of walking, the walking speed of the subject 10 based on the walking time and the walking distance.

The qualitative measurement value acquirer 40 also receives, via the communication unit 52, information measured by the measurement device 20 worn by the subject 10. Based on the information received from the measurement device 20, the qualitative measurement value acquirer 40 acquires a measurement value of at least one of the stride length, cadence, arm swing angle, pelvic movement, difference between the left and right walking tempos, and the like, as a qualitative parameter indicating the walking form. The qualitative measurement value acquirer 40 includes a stride length acquirer 42, a cadence acquirer 44, an arm movement acquirer 46, a hip movement acquirer 48, and a tempo acquirer 49.

The stride length acquirer 42 acquires, as a qualitative parameter indicating the walking form, the stride length of the subject 10 based on the information received from the position measurement unit 24 and the motion detector 26. The stride length as used herein may be an average value of the stride length per unit time or unit distance. The cadence acquirer 44 acquires, as a qualitative parameter indicating the walking form, the cadence of the subject 10 based on the information received from the motion detector 26. The cadence as used herein may be an average value of the cadence per unit time or unit distance.

The arm movement acquirer 46 acquires, as a qualitative parameter indicating the walking form, the arm swing angle of the subject 10 based on the information received from the motion detector 26. Also, the hip movement acquirer 48 acquires, as a qualitative parameter indicating the walking form, the pelvic movement of the subject 10 based on the information received from the motion detector 26. The measurement values of the "pelvic movement" as used herein may include the pelvic or hip rotation angle, lateral tilt angle of the pelvis or hips, and front-back bend angle of the pelvis or hips. The tempo acquirer 49 acquires, as a qualitative parameter indicating the walking form, the difference between the left and right walking tempos of the subject 10 based on the information received from the motion detector 26. The "difference between the left and right walking tempos" as used herein may be the difference between the cadence interval from the left foot to the right foot and the cadence interval from the right foot to the left foot.

The output unit 51 displays, on the screen, the measurement values of the quantitative parameters (walking time, walking distance, and walking speed) indicating the amount of walking acquired by the quantitative measurement value acquirer 30 and the measurement values of the qualitative parameters (stride length, cadence, arm swing angle, pelvic movement, and difference between the left and right walking tempos) indicating the walking form acquired by the qualitative measurement value acquirer 40. The output unit 51 may display both the measurement values of the quantitative parameters and the measurement values of the qualitative parameters on the screen while the subject 10 is performing walking exercise or may display only the quantitative parameters on the screen during the walking exercise. Also, the output unit 51 may display the measurement values of the quantitative parameters and the measurement values of the qualitative parameters on the screen after the completion of the walking exercise. The output unit 51 transmits the measurement values of the quantitative parameters and the measurement values of the qualitative parameters as the walking log data together with the attribute information of the subject 10, to the information management server 60 via the communication unit 52. The output unit 51 may transmit, as the walking log, the measurement values of the time information, position information, number of steps per unit time, arm swing angle, and the like acquired from the measurement device 20 to the information management server 60 or may further transmit, together with the measurement values acquired from the measurement device 20, information such as the walking time, walking distance, walking speed, stride length, and cadence, as the walking log to the information management server 60.

The information management server 60 includes a communication unit 62, a log acquirer 64, a quantitative measurement value acquirer 70, a qualitative measurement value acquirer 80, a result determination unit 90, a data storage unit 98, and an output unit 99. The log acquirer 64 acquires the walking log data from the information terminal 50 via the communication unit 62 and accumulates the walking log data in the data storage unit 98.

Based on the walking log data acquired by the log acquirer 64, the quantitative measurement value acquirer 70 acquires a measurement value of a quantitative parameter indicating the amount of walking, such as the walking time, walking distance, and the walking speed. The quantitative measurement value acquirer 70 includes a time acquirer 72, a distance acquirer 74, and a speed acquirer 76.

The time acquirer 72 acquires, as a quantitative parameter indicating the amount of walking, the walking time of the subject 10 based on the walking log data. The distance acquirer 74 acquires, as a quantitative parameter indicating the amount of walking, the walking distance of the subject 10 based on the walking log data. The speed acquirer 76 acquires, as a quantitative parameter indicating the amount of walking, the walking speed of the subject 10 based on the walking log data.

Based on the walking log data acquired by the log acquirer 64, the qualitative measurement value acquirer 80 acquires a measurement value of at least one of the stride length, cadence, arm swing angle, pelvic movement, left and right walking tempos, and the like, as a qualitative parameter indicating the walking form. The qualitative measurement value acquirer 80 includes a stride length acquirer 82, a cadence acquirer 84, an arm movement acquirer 86, a hip movement acquirer 88, and a tempo acquirer 89.

The stride length acquirer 82 acquires, as a qualitative parameter indicating the walking form, the stride length of the subject 10 based on the walking log data. The cadence acquirer 84 acquires, as a qualitative parameter indicating the walking form, the cadence of the subject 10 based on the walking log data. The arm movement acquirer 86 acquires, as a qualitative parameter indicating the walking form, the arm swing angle of the subject 10 based on the walking log data. The hip movement acquirer 88 acquires, as a qualitative parameter indicating the walking form, the pelvic movement of the subject 10 based on the walking log data. The tempo acquirer 89 acquires, as a qualitative parameter indicating the walking form, the difference between the left and right walking tempos of the subject 10 based on the walking log data.

The result determination unit 90 includes an attribute acquirer 92, a reference value calculation unit 93, a quantitative result determination unit 94, a qualitative result determination unit 96, and a condition judgment unit 97.

The attribute acquirer 92 acquires the attribute information of the subject 10 from the data storage unit 98, based on the walking log data or the identification information of the subject 10 related to the walking log data. The attribute information includes, for example, information such as the age, gender, and height of the subject 10 and information on a level regarding physical strength or health to which the subject 10 belongs. The physical strength level is based on measurement values obtained by comprehensive measurement of muscle strength, flexibility (the ranges of motion of the hip joints and the shoulder joints), grip strength, leg strength (sit-to-stand test), balance strength (one-leg standing with eyes closed), endurance, and the like. The physical strength level may also include other physical strength levels evaluated for measurement items in physical strength evaluation commonly used. The health level is based on measurement values of, for example, indices used in medical examinations (height, weight, BMI, blood pressure, blood indices) and a stress index.

The reference value calculation unit 93 calculates average measurement values for each attribute or level to which the subject 10 belongs, as reference values to be compared with various measurement values of the subject 10. The reference value calculation unit 93 may calculate average measurement values for each generation, gender, physical strength level, and health level, for example.

The quantitative result determination unit 94 determines an evaluation result regarding the amount of walking, based on the measurement values of quantitative parameters acquired by the quantitative measurement value acquirer 70. The quantitative result determination unit 94 may determine the evaluation result based on the measurement values of quantitative parameters such as the walking time, walking distance, and walking speed, in comparison with the average values thereof for each attributed level. For example, the case where the walking time is longer than the corresponding generation's average thereof may be evaluated as "a larger amount of exercise", and the case where the walking time is shorter than the corresponding generation's average thereof may be evaluated as "a smaller amount of exercise".

The qualitative result determination unit 96 determines an evaluation result regarding the walking form, based on the measurement values of qualitative parameters acquired by the qualitative measurement value acquirer 80 and a predetermined evaluation model that reflects the degree of approximation to a form tendency with which a walking exercise effect is likely to be achieved. As the predetermined evaluation model that reflects the degree of approximation to a form tendency with which a walking exercise effect is likely to be achieved, an evaluation formula can be considered, for example, in which, in the case where a better value of a specific qualitative parameter leads closer to an ideal form for a higher exercise effect, the weighting factor for the specific qualitative parameter is made larger so that the qualitative parameter is reflected more in the evaluation. Also, a different value may be set as the weighting factor for each qualitative parameter so as to be proportional to the magnitude of contribution to the exercise effect.

The qualitative result determination unit 96 determines the evaluation result regarding the walking form using, as the predetermined evaluation model, an evaluation formula set such that a weight is added at least to the measurement value of the stride length. The qualitative result determination unit 96 may determine the evaluation result regarding the walking form using, as the predetermined evaluation model, an evaluation formula for obtaining the sum of a value obtained by adding a weight to the ratio of the stride-height ratio of the subject 10 to the average stride-height ratio of the corresponding attributed level, and the value of the ratio of the cadence of the subject 10 to the average cadence of the corresponding attributed level. The qualitative result determination unit 96 may also determine the evaluation result regarding the walking form using, as the predetermined evaluation model, an evaluation formula set such that a weight is added at least to the measurement value of the pelvic movement. The qualitative result determination unit 96 may also determine the evaluation result regarding the walking form using, as the predetermined evaluation model, an evaluation formula set such that a weight is added at least to the measurement value of the arm swing angle. The evaluation model or the evaluation formula will be described later.

The condition judgment unit 97 judges whether or not at least one of the evaluation result regarding the amount of walking or the evaluation result regarding the walking form has satisfied a predetermined target achievement condition. In recent years, a method called gamification has been used to continue exercise habits for the purpose of improving health through exercise. For example, a game-like method can be considered in which, in addition to the calculation of energy consumption based on the walking distance and the number of steps taken and to the estimation and display of the amount of body fat reduction, a specific game is made to progress each time a certain condition is met, and discount tickets or points are issued based on the degree of achievement of the game. Thus, the condition judgment unit 97 judges whether or not any of multiple types of target achievement conditions, presented as such game-like elements to the subject 10, has been satisfied.

A target achievement condition judged by the condition judgment unit 97 may be, for example, a condition of awarding 1 point for each time the walking distance reaches 1 km, or a condition of awarding 1 point for each time the number of steps reaches 100 steps. Also, while the point award rate is usually one times, it may be increased to 1.1 times or the like for each time a favorable result is obtained as the evaluation result regarding the walking form. Other target achievement conditions may be, for example, "to walk for 30 minutes or longer five times", "to obtain a favorable result as the exercise effect on the thighs five times", "to walk 3 km or longer once", "to walk at an average walking speed of 6 km/h five times", "to obtain a favorable result as the exercise effect on the vicinity of the waist five times", "to obtain a favorable result as the exercise effect on the upper arms five times", "to walk 10 km once", "to perform walking exercise 10 times", and so on. Also, these various target achievement conditions may be displayed in a 3x3 matrix on the screen such that the game is cleared when targets for the squares of any of the horizontal, vertical, or diagonal rows are achieved, just like a bingo game.

The output unit 99 outputs walking result information including the evaluation result regarding the amount of walking and the evaluation result regarding the walking form, to the information terminal 50 via the communication unit 62. The output unit 99 outputs the walking result information including information regarding whether or not at least one of the evaluation result regarding the amount of walking or the evaluation result regarding the walking form has satisfied a predetermined target achievement condition, to the information terminal 50 via the communication unit 62. By allowing the output unit 99 to display the information regarding whether or not a target achievement condition has been satisfied on the screen of the information terminal 50, the subject 10 can be motivated to exercise and also encouraged to continue the exercise.

The output unit 99 outputs the walking result information including information regarding the exercise effect on each body part based on the evaluation result regarding the amount of walking and the evaluation result regarding the walking form, to the information terminal 50 via the communication unit 62. For example, the output unit 99 may display, on the screen of the information terminal 50, an object indicating the type and magnitude of the exercise effect on each body part on an image of a human body model to express the exercise effect on each body part.

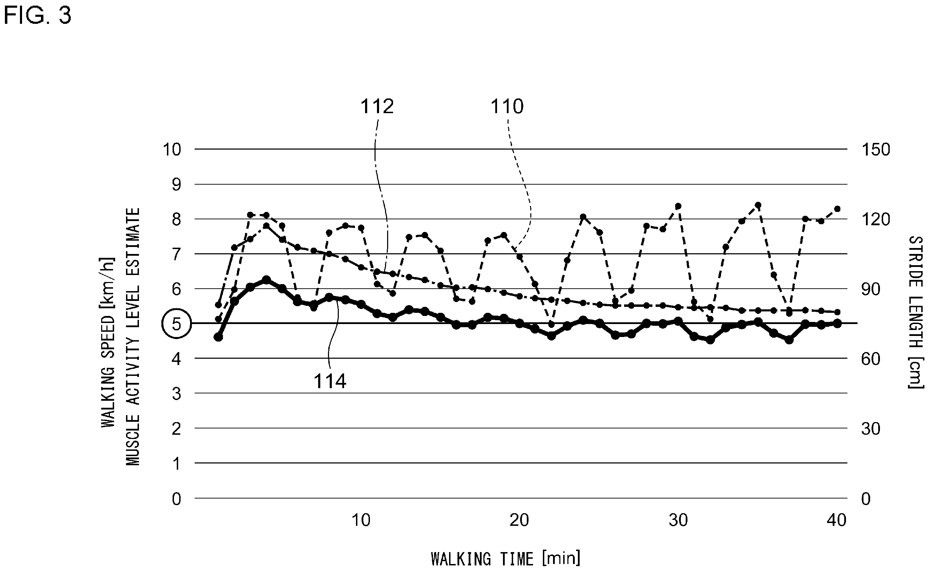

FIG. 3 shows relationships between the walking speed, stride length, and muscle activity level. The muscle activity level is an example of a qualitative evaluation value for walking exercise estimated by the qualitative result determination unit 96 with a predetermined evaluation model. It can be said that the larger the amount of muscle activity in each body part, the higher the exercise effect on each body part. For example, in the case of the so-called cadence type, of which the cadence is relatively large, it can be said that the range of motion of the legs is relatively narrow, the leg muscles are not sufficiently stretched, and the amount of muscle activity is relatively small. Conversely, in the case of the so-called stride type, of which the stride length is relatively large, it can be said that the range of motion of the legs is relatively wide, the leg muscles are sufficiently stretched, and the amount of muscle activity is relatively large. Accordingly, it is considered that the load on the leg muscles is greater in walking with a larger range of motion and a smaller number of repetitions than in walking with a smaller range of motion and a larger number of repetitions. Therefore, with reference to FIG. 3, the muscle activity level of which the relationship with the stride length was particularly evaluated greatly will be described.

FIG. 3 shows the results of interval walking performed by the subject 10, in which walking at a speed increased to a point where it is not regarded as running (also called fast walking) and walking at a slower speed were repeated alternately about every 3 minutes. Such interval walking is known to provide exercise effects comparable to those of running, while placing less strain on the legs than running. In the graph of FIG. 3, the horizontal axis represents the walking time [minutes]. Also, the right vertical axis represents the stride length [cm], and the left vertical axis represents the walking speed [km/h] and the muscle activity level estimate.

In the graph of FIG. 3, the line of walking speed 110 indicates an average walking speed for each minute. The line of walking speed 110 increases to about 7-8 km/h during fast walking and decreases to about 5-6 km/h during slow walking in the intervals, showing such an increase and decrease in speed repeated about every 3 minutes. The line of stride length 112 indicates an average stride length for each minute. The line of stride length 112 indicates walking with a large stride length of about 100-120 cm immediately after the start and, over the walking time, the stride length gradually decreasing to about 80 cm. The line of muscle activity level estimation 114 indicates an average muscle activity level estimate for each minute. The line of muscle activity level estimation 114 indicates that the muscle activity level estimate increases to around 6 immediately after the start and, while increasing and decreasing along with the increase or decrease of the walking speed 110, gradually decreases to about 4.5 to 5 over the walking time.

The qualitative evaluation values for walking exercise can be divided into multiple types of evaluation values for each body part, and the exercise effect can be measured for each body part. This also enables measurement of an exercise effect in terms of dieting focusing on a specific target body part, which may also be called "localized slimming". In the following, a method for estimating an evaluation value for each body part will be described.

Muscle Activity Level in Buttocks and Thighs

The line of muscle activity level estimation 114 in the graph of FIG. 3 indicates an evaluation value of the muscle activity level in the buttocks and thighs. Normally, the amount of activity of the buttock and thigh muscle groups, such as the gluteus maximus, hamstrings, and rectus femoris, is proportional to the walking speed. The walking speed is thought to be more influenced by the stride length than by the cadence. The muscle activity level in the buttocks and thighs can be estimated from the two variables of the stride length and the cadence. A muscle activity level estimate y per minute is determined by the qualitative result determination unit 96 using the following estimation formula.

$$y = 4*a/A + 1*b/B$$

where a represents the stride-height ratio per minute, b represents the cadence per minute, A represents the average stride-height ratio of the corresponding generation, and B represents the average cadence of the corresponding generation. The "stride-height ratio" indicates the ratio of the stride length to the height of the subject 10. For the weighting such that the degree of contribution of the stride length becomes greater than that of the cadence, the weighting factor for the stride length may be set to 4, and the weighting factor for the cadence may be set to 1, for example.

For example, if the measurement values of the stride length and the cadence are equal to the corresponding generation's averages thereof, a/A and b/B will be both 1 and hence y = 5, which corresponds to the reference value. In the graph of FIG. 3, the scale of "5" located in the middle of the left vertical axis indicates the reference value, and, when the evaluation value is positioned above the reference value, i.e., when the estimate y is greater than 5 as the reference value, the muscle activity level in the buttocks and thighs of the subject 10 can be evaluated as high. When the evaluation value is positioned below the reference value, i.e., when the estimate y is smaller than 5 as the reference value, the muscle activity level in the buttocks and thighs of the subject 10 can be evaluated as low.

Muscle Activity Level in the Vicinity of Waist

Normally, the amount of activity of the muscle groups around the waist, such as the rectus abdominis muscles and oblique abdominal muscles, is increased by rotating the pelvis greatly during walking. The muscle activity level in the vicinity of the waist can be estimated from the two variables of the pelvic rotation angle and the cadence. The muscle activity level estimate y per minute is determined by the qualitative result determination unit 96 using the following estimation formula.

$$y = 4*c/C + 1*b/B$$

where c represents the average pelvic rotation angle per minute, b represents the cadence per minute, C represents an average pelvic rotation angle, and B represents the average cadence of the corresponding generation. For the weighting such that the degree of contribution of the pelvic rotation angle becomes greater than that of the cadence, the weighting factor for the pelvic rotation angle may be set to 4, and the weighting factor for the cadence may be set to 1, for example.

For example, if the measurement values of the pelvic rotation angle and the cadence are equal to the corresponding generation's averages thereof, c/C and b/B will be both 1 and hence y = 5, which corresponds to the reference value. When the estimate y is greater than 5 as the reference value, the muscle activity level in the vicinity of the waist of the subject 10 can be evaluated as high. When the estimate y is smaller than 5 as the reference value, the muscle activity level in the vicinity of the waist of the subject 10 can be evaluated as low.

While the pelvic rotation angle has a high degree of contribution to the estimation of the muscle activity level in the vicinity of the waist, the measurement values of the lateral hip tilt and the front-back hip bend have no direct effect on the estimation of the muscle activity level in the vicinity of the waist and have a relatively low degree of contribution. However, the measurement values of the lateral hip tilt and the front-back hip bend may be added, as variables with relatively low weighting factors, to the evaluation formula for the muscle activity level in the vicinity of the waist so that the degree of approximation to a form tendency with which a walking exercise effect is likely to be achieved can be measured more accurately.

Muscle Activity Level in Upper Arms

Normally, the amount of activity of the upper arm muscle groups, such as the biceps and triceps brachii muscles, is increased by swinging the arms greatly during walking. The muscle activity level in the upper arms can be estimated from the two variables of the arm swing angle and the cadence. The muscle activity level estimate y per minute is determined by the qualitative result determination unit 96 using the following estimation formula.

$$y = 4*d/D + 1*b/B$$

where d represents the average arm swing angle per minute, b represents the cadence per minute, D represents an average arm swing angle, and B represents the average cadence of the corresponding generation. For the weighting such that the degree of contribution of the arm swing angle becomes greater than that of the cadence, the weighting factor for the arm swing angle may be set to 4, and the weighting factor for the cadence may be set to 1, for example.

For example, if the measurement values of the arm swing angle and the cadence are equal to the corresponding generation's averages thereof, diD and b/B will be both 1 and hence y = 5, which corresponds to the reference value. When the estimate y is greater than 5 as the reference value, the muscle activity level in the upper arms of the subject 10 can be evaluated as high. When the estimate y is smaller than 5 as the reference value, the muscle activity level in the upper arms of the subject 10 can be evaluated as low.

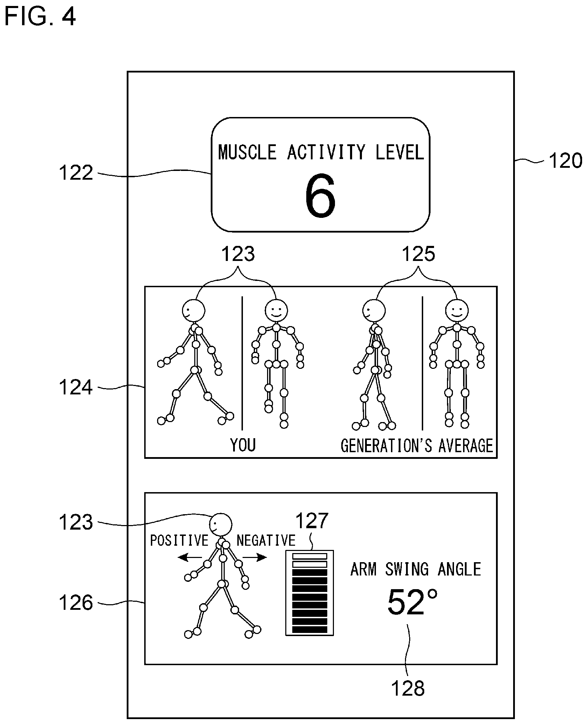

FIG. 4 illustrates a screen example for displaying the muscle activity level estimate. A screen 120 shows the contents displayed on the screen of the information terminal 50. In a first frame 122, the number "6" is displayed as the muscle activity level estimate determined by the qualitative result determination unit 96. In a second frame 124, the ratio of the stride length to the average stride length of the corresponding generation is represented by a stick figure object indicating the magnitude of the muscle activity level with the skeleton of a person performing walking exercise. As shown in FIG. 4, a first stick figure 123 representing the subject 10 is displayed at a skeletal position such that the magnitude of the muscle activity level is different from that of a second stick figure 125 representing the corresponding generation's average, thereby visually indicating that the subject 10 exceeds the generation's average. The first stick figure 123 and the second stick figure 125 may be displayed in animated forms performing walking exercise, and, in that case, the magnitude of each of the arm swing, leg movement, and hip sway on the left and right sides may be expressed by the magnitude of the skeletal movement in the animation. Also, in the first stick figure 123, part of the body parts mainly evaluated for the muscle activity level may be emphasized with a specific color. In a third frame 126, the measurement value of the arm swing angle is displayed with a level indicator 127 and a text 128. As shown in FIG. 4, the level indicated by the level indicator 127 is the sixth of the total eight levels, which visually indicates that the measurement value is greater than a standard value as an intermediate value.

On the screen shown in FIG. 4, the arm swing angle is displayed as being mainly evaluated, thereby indicating that the muscle activity level estimate displayed in the first frame 122 and the second frame 124 is based mainly on the evaluation of the muscle activity level in the upper arms. In another aspect, when the muscle activity level in the buttocks and thighs is mainly evaluated, it may be indicated in the third frame 126 that it is mainly the result of evaluation of the stride length, for example. Also, in another aspect, when the muscle activity level in the vicinity of the waist is mainly evaluated, it may be indicated in the third frame 126 that it is mainly the result of evaluation of the average pelvic rotation angle, for example.

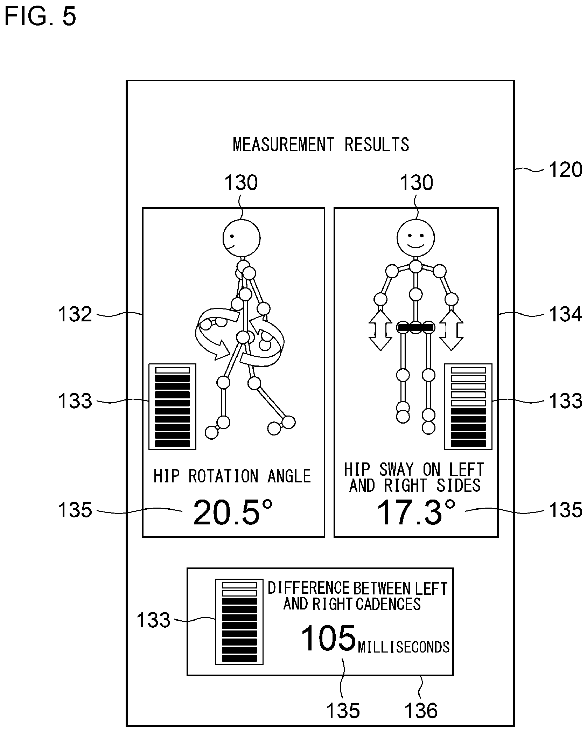

FIG. 5 illustrates a screen example for displaying measurement values as qualitative parameters. The screen 120 shows the contents displayed on the screen of the information terminal 50. On the screen 120, a fourth frame 132, a fifth frame 134, and a sixth frame 136 are arranged. In the fourth frame 132, a third stick figure 130 with arrows, which indicate hip rotation, is displayed, together with a level indicator 133 that indicates the hip rotation level, and with a text 135 that indicates the measurement value of the hip rotation angle (a numerical value such as "20.5 degrees", for example). In the fifth frame 134, the third stick figure 130 is displayed with arrows indicating hip sway on the left and right sides, the level indicator 133 indicates the sway level, and the text 135 indicates the measurement value of the lateral pelvic tilt angle (a numerical value such as "17.3 degrees", for example). In the sixth frame 136, the level indicator 133 indicates the level of the difference between the left and right cadences, and the text 135 indicates the measurement value of the difference between the left and right cadences (a numerical value such as "105 milliseconds", for example). In the same way as shown in FIG. 5, the measurement value of the arm swing angle, the measurement value of the front-back bend angle of the pelvis, and the measurement value of the stride length or cadence may be further displayed.

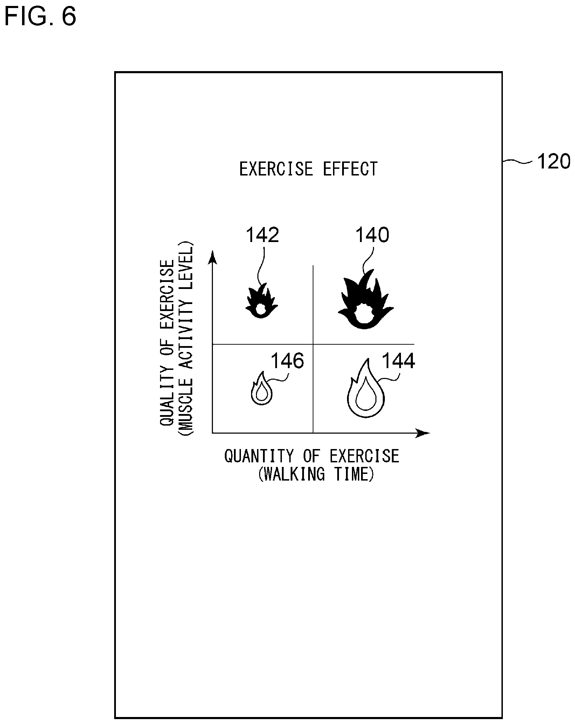

FIG. 6 illustrates a screen example for displaying exercise effects divided based on a quantitative parameter and a qualitative parameter. The screen 120 shows the contents displayed on the screen of the information terminal 50. In the graph of FIG. 6, the horizontal axis represents the quantity of exercise, i.e., the walking time as the amount of walking, and the vertical axis represents the quality of exercise, i.e., the muscle activity level. In the matrix of the graph, a first effect object 140 located on the upper right is a large flame mark representing complete combustion, which indicates that both the quantity and the quality of exercise are high. A second effect object 142 located on the upper left is a small flame mark representing complete combustion, which indicates that the quantity of exercise is low but the quality of exercise is high. A third effect object 144 located on the lower right is a large flame mark representing incomplete combustion, which indicates that the quantity of exercise is high but the quality of exercise is low. A fourth effect object 146 located on the lower left is a small flame mark representing incomplete combustion, which indicates that both the quantity and the quality of exercise are low. On the actual screen, one of the first effect object 140, second effect object 142, third effect object 144, or fourth effect object 146 is displayed to visually indicate the quantity and quality of exercise such as to be grasped at a glance.

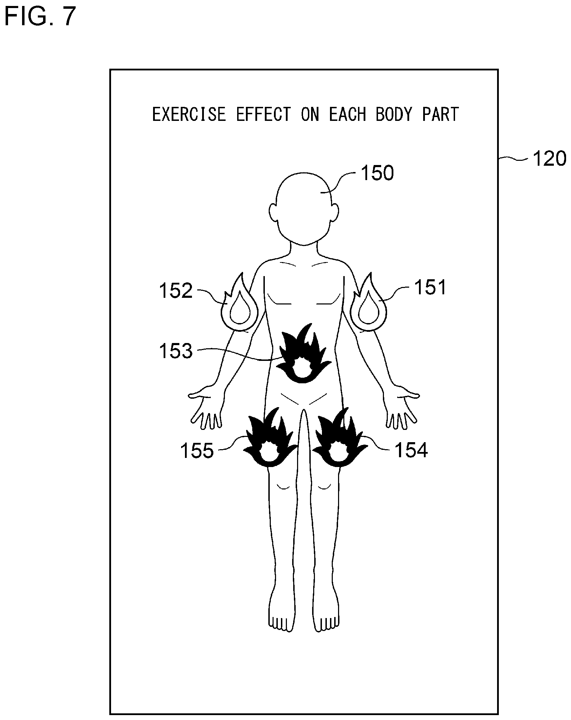

FIG. 7 illustrates a screen example for displaying an exercise effect on each body part. The screen 120 shows the contents displayed on the screen of the information terminal 50. In the screen example of FIG. 7, a human body model 150 is displayed in the center of the screen, and a flame mark object is displayed for each body part. For example, a fifth effect object 151 and a sixth effect object 152 are displayed at positions corresponding to the left and right upper arms. The fifth effect object 151 and the sixth effect object 152 are large flame marks representing incomplete combustion, similar to the third effect object 144 shown in FIG. 6. This visually indicates that, with regard to the left and right upper arms, the exercise effect was high in the quantity of exercise but low in the quality of exercise. Also, a seventh effect object 153 is displayed at a position around the waist, for example. The seventh effect object 153 is a large flame mark representing complete combustion, similar to the first effect object 140 shown in FIG. 6. This visually indicates that, with regard to the vicinity of the waist, the exercise effect was high in both the quantity and the quality of exercise. Also, an eighth effect object 154 and a ninth effect object 155 are displayed at positions of the left and right thighs, for example. The eighth effect object 154 and the ninth effect object 155 are large flame marks representing complete combustion, similar to the first effect object 140 shown in FIG. 6. This visually indicates that, with regard to the thighs, the exercise effect was high in both the quantity and the quality of exercise. Further, when the quantity of exercise is low, an effect object similar to the second effect object 142 or the fourth effect object 146 shown in FIG. 6 may be displayed on the target body part. This allows the effect in the quantity and quality of exercise for each body part to be grasped at a glance.

The present invention is defined by the claims.

**Claims**

1.   A gait evaluation system (100), comprising:

   a quantitative measurement value acquirer (40, 80) that acquires a measurement value of a quantitative parameter indicating an amount of walking, measured by a measurement device (20) worn by a subject;
   a qualitative measurement value acquirer that acquires at least measurement values of a stride length, of an arm swing angle and a cadence as qualitative parameters indicating a walking form, measured by a measurement device (20) worn by the subject;
   a quantitative result determination unit that determines an evaluation result regarding an amount of walking, based on a measurement value of the quantitative parameter;
   a qualitative result determination unit (96) that determines an evaluation result regarding a walking form, based on a predetermined evaluation model that reflects the degree of approximation to a form tendency with which a walking exercise effect is likely to be achieved, and measurement values of the qualitative parameters; and
   an output unit that outputs walking result information including the evaluation result regarding the amount of walking and the evaluation result regarding the walking form,
   wherein the qualitative result determination unit (96) determines the evaluation result regarding the walking form using, as the predetermined evaluation model, an evaluation formula for obtaining the sum of a value obtained by adding a weight to the ratio of the subject's stride-height ratio to an average stride-height ratio of a corresponding attributed level, and a value of the ratio of the subject's cadence to an average cadence of the corresponding attributed level, and an evaluation formula that is set such that a weight is added at least to a measurement value of the arm swing angle.

2.   The gait evaluation system (100) according to claim 1, wherein the qualitative result determination unit (96) determines the evaluation result regarding the walking form using, as the predetermined evaluation model, an evaluation formula that is set such that a weight is added at least to a measurement value of the stride length.

3.   The gait evaluation system (100) according to claim 1 or 2, wherein the measurement value of the stride length is a ratio of an average stride length to the height of the subject measured.

4.   The gait evaluation system (100) according to any one of claims 1 through 3, wherein

   the qualitative measurement value acquirer further acquires a measurement value of pelvic movement as the qualitative parameters, and
   the qualitative result determination unit (96) determines the evaluation result regarding the walking form using, as the predetermined evaluation model, an evaluation formula that is set such that a weight is added at least to a measurement value of the pelvic movement.

5.   The gait evaluation system (100) according to any one of claims 1 through 4, wherein the output unit outputs the walking result information including information regarding whether or not at least one of the evaluation result regarding the amount of walking or the evaluation result regarding the walking form has satisfied a predetermined target achievement condition.

6.   The gait evaluation system (100) according to any one of claims 1 through 5, wherein the output unit outputs the walking result information including information regarding an exercise effect on each body part based on the evaluation result regarding the amount of walking and the evaluation result regarding the walking form.

7.   A gait evaluation system (100), comprising:

   a quantitative measurement value acquirer (40, 80) that acquires a measurement value of a quantitative parameter indicating an amount of walking, measured by a measurement device (20) worn by a subject;
   a qualitative measurement value acquirer that acquires at least measurement values of pelvic movement, of an arm swing angle and a cadence as qualitative parameters indicating a walking form, measured by a measurement device (20) worn by the subject;
   a quantitative result determination unit that determines an evaluation result regarding an amount of walking, based on a measurement value of the quantitative parameter;

a qualitative result determination unit (96) that determines an evaluation result regarding a walking form, based on a predetermined evaluation model that reflects the degree of approximation to a form tendency with which a walking exercise effect is likely to be achieved, and measurement values of the qualitative parameters; and

an output unit that outputs walking result information including the evaluation result regarding the amount of walking and the evaluation result regarding the walking form,

wherein the qualitative result determination unit (96) determines the evaluation result regarding the walking form using, as the predetermined evaluation model, an evaluation formula for obtaining the sum of a value obtained by adding a weight to the ratio of the subject's stride-height ratio to an average stride-height ratio of a corresponding attributed level, and a value of the ratio of the subject's cadence to an average cadence of the corresponding attributed level, and an evaluation formula that is set such that a weight is added at least to a measurement value of the arm swing angle.

8. A gait evaluation method, comprising:

acquiring a measurement value of a quantitative parameter indicating an amount of walking, measured by a measurement device (20) worn by a subject;

acquiring at least measurement values of a stride length, of an arm swing angle and a cadence as qualitative parameters indicating a walking form, measured by a measurement device (20) worn by the subject;

determining an evaluation result regarding an amount of walking, based on a measurement value of the quantitative parameter;

determining an evaluation result regarding a walking form, based on a predetermined evaluation model that reflects the degree of approximation to a form tendency with which a walking exercise effect is likely to be achieved, and measurement values of the qualitative parameters; and

outputting walking result information including the evaluation result regarding the amount of walking and the evaluation result regarding the walking form,

wherein, in determining the evaluation result regarding a walking form, further determining the evaluation result regarding the walking form using, as the predetermined evaluation model, an evaluation formula for obtaining the sum of a value obtained by adding a weight to the ratio of the subject's stride-height ratio to an average stride-height ratio of a corresponding attributed level, and a value of the ratio of the subject's cadence to an average cadence of the corresponding attributed level, and an evaluation formula that is set such that a weight is added at least to a measurement value of the arm swing angle.

**Patentansprüche**

1. Gangbewertungssystem (100), mit:

einem quantitativen Messwerterfasser (40, 80), der einen Messwert eines quantitativen Parameters bezieht, der einen Betrag des Gehens anzeigt, gemessen von einer Messvorrichtung (20), die von einer Person getragen wird;

einem qualitativen Messwerterfasser, der zumindest Messwerte einer Schrittlänge, eines Armschwingungswinkels und einer Kadenz als qualitative Parameter erfasst, die eine Gangform angeben, die von einer von der Person getragenen Messvorrichtung (20) gemessen werden;

einer quantitativen Ergebnisbestimmungseinheit, die auf der Grundlage eines Messwerts des quantitativen Parameters ein Bewertungsergebnis bezüglich eines Betrags des Gehens bestimmt;

einer qualitativen Ergebnisbestimmungseinheit (96), die ein Bewertungsergebnis bezüglich einer Gangform bestimmt, basierend auf einem vorbestimmten Bewertungsmodell, das den Grad der Annäherung an eine Formtendenz widerspiegelt, mit der ein Gehübungseffekt wahrscheinlich erreicht wird, und Messwerten der qualitativen Parameter; und

einer Ausgabeeinheit, die Gangergebnisinformationen ausgibt, die das Bewertungsergebnis bezüglich des Betrags des Gehens und das Bewertungsergebnis bezüglich der Gangform umfassen,

wobei die qualitative Ergebnisbestimmungseinheit (96) das Bewertungsergebnis bezüglich der Gangform bestimmt, indem als vorbestimmtes Bewertungsmodell eine Bewertungsformel verwendet wird, um die Summe eines Wertes zu erhalten, der erhalten wird, indem eine Gewichtung zu dem Verhältnis des Schritthöhenverhältnisses des Subjekts zu einem durchschnittlichen Schritthöhenverhältnis eines entsprechenden zugewiesenen Niveaus addiert wird, und eines Wertes des Verhältnisses der Trittfrequenz des Subjekts zu einer durchschnittlichen Trittfrequenz des entsprechenden zugewiesenen Niveaus, und einer Bewertungsformel, die so eingestellt ist, dass eine Gewichtung zumindest zu einem Messwert des Armschwingwinkels addiert wird.

2. Gangbewertungssystem (100) gemäß Anspruch 1, wobei die qualitative Ergebnisbestimmungseinheit (96) das Bewertungsergebnis bezüglich der Gangform bestimmt, indem als vorbestimmtes Bewertungsmodell eine Bewertungsformel verwendet wird, die so eingestellt ist, dass eine Gewichtung mindestens zu einem Messwert der Schrittlänge addiert wird.

3. Gangbewertungssystem (100) gemäß Anspruch 1 oder 2, wobei der Messwert der Schrittlänge ein Verhältnis einer durchschnittlichen Schrittlänge zur Körpergröße der gemessenen Person ist.

4. Gangbewertungssystem (100) gemäß einem der Ansprüche 1 bis 3, wobei

der Erfasser der qualitativen Messwerterfasser weiterhin einen Messwert der Beckenbewegung als die qualitativen Parameter erfasst, und die qualitative Ergebnisbestimmungseinheit (96) das Bewertungsergebnis bezüglich der Gangform bestimmt, indem als vorbestimmtes Bewertungsmodell eine Bewertungsformel verwendet wird, die so eingestellt ist, dass zumindest zu einem Messwert der Beckenbewegung eine Gewichtung addiert wird.

5. Gangbewertungssystem (100) gemäß einem der Ansprüche 1 bis 4, wobei die Ausgabeeinheit die Gangergebnisinformationen ausgibt, die Informationen darüber enthalten, ob mindestens eines der Bewertungsergebnisse bezüglich des Gehens oder des Bewertungsergebnisses bezüglich der Gangform eine vorbestimmte Zielerreichungsbedingung erfüllt hat oder nicht.

6. Gangbewertungssystem (100) gemäß einem der Ansprüche 1 bis 5, wobei die Ausgabeeinheit die Gangergebnisinformationen ausgibt, die Informationen bezüglich eines Trainingseffekts auf jeden Körperteil auf der Grundlage des Bewertungsergebnisses bezüglich des Betrag des Gehens und des Bewertungsergebnisses bezüglich der Gangform enthalten.

7. Gangbewertungssystem (100), mit:

einem quantitativen Messwerterfasser (40, 80), der einen Messwert eines quantitativen Parameters erfasst, der einen Betrag des Gehens anzeigt, gemessen von einer Messvorrichtung (20), die von einer Person getragen wird;
einem qualitativen Messwerterfasser, der zumindest Messwerte der Beckenbewegung, eines Armschwingungswinkels und einer Kadenz als qualitative Parameter erfasst, die eine Gangform anzeigen, die von einer von der Person getragenen Messvorrichtung (20) gemessen werden;
einer quantitativen Ergebnisbestimmungseinheit, die auf der Grundlage eines Messwerts des quantitativen Parameters ein Auswertungsergebnis bezüglich eines Gehens bestimmt;
einer qualitativen Ergebnisbestimmungseinheit (96), die ein Bewertungsergebnis bezüglich einer Gangform bestimmt, basierend auf einem vorbestimmten Bewertungsmodell, das den Grad der Annäherung an eine Formtendenz widerspiegelt, mit der ein Gehübungseffekt wahrscheinlich erreicht wird, und Messwerten der qualitativen Parameter; und
einer Ausgabeeinheit, die Gangergebnisinformationen ausgibt, die das Bewertungsergebnis bezüglich des Laufumfangs und das Bewertungsergebnis bezüglich der Gangform umfassen,
wobei die qualitative Ergebnisbestimmungseinheit (96) das Bewertungsergebnis bezüglich der Gangform bestimmt, indem als das vorbestimmte Bewertungsmodell eine Bewertungsformel verwendet wird, um die Summe eines Wertes, der durch Addieren einer Gewichtung zu dem Verhältnis des Schritthöhenverhältnisses des Subjekts zu einem durchschnittlichen Schritthöhenverhältnis eines entsprechenden zugewiesenen Niveaus erhalten wird, und eines Wertes des Verhältnisses der Trittfrequenz des Subjekts zu einer durchschnittlichen Trittfrequenz des entsprechenden zugewiesenen Niveaus und einer Bewertungsformel zu erhalten, die so eingestellt ist, dass eine Gewichtung zumindest zu einem Messwert des Armschwingwinkels addiert wird.

8. Gangbewertungsverfahren, mit:

Erfassen eines Messwerts eines quantitativen Parameters, der einen Betrag des Gehens anzeigt, gemessen durch eine Messvorrichtung (20), die von einer Person getragen wird;
Erfassen zumindest von Messwerten einer Schrittlänge, eines Armschwingungswinkels und einer Kadenz als qualitative Parameter, die eine Gangform anzeigen, gemessen durch eine von der Person getragene Messvorrichtung (20);
Bestimmen eines Bewertungsergebnisses bezüglich eines Gehens, basierend auf einem Messwert des quantitativen Parameters;
Bestimmen eines Bewertungsergebnisses bezüglich einer Gangform, basierend auf einem vorbestimmten Bewertungsmodell, das den Grad der Annäherung an eine Formtendenz widerspiegelt, mit der ein Gehübungseffekt wahrscheinlich erreicht wird, und Messwerten der qualitativen Parameter; und

Ausgeben von Gangergebnisinformationen einschließlich des Bewertungsergebnisses bezüglich des Gehumfangs und des Bewertungsergebnisses bezüglich der Gangform,

wobei beim Bestimmen des Bewertungsergebnisses bezüglich einer Gangform ferner das Bewertungsergebnis bezüglich der Gangform unter Verwendung einer Bewertungsformel als das vorbestimmte Bewertungsmodell bestimmt wird, um die Summe eines Wertes, der durch Addieren einer Gewichtung zu dem Verhältnis des Schritthöhenverhältnisses des Subjekts zu einem durchschnittlichen Schritthöhenverhältnis eines entsprechenden zugeordneten Niveaus erhalten wird, und eines Wertes des Verhältnisses der Trittfrequenz des Subjekts zu einer durchschnittlichen Trittfrequenz des entsprechenden zugeordneten Niveaus und einer Bewertungsformel zu erhalten, die so eingestellt ist, dass eine Gewichtung zumindest zu einem Messwert des Armschwingwinkels addiert wird.

**Revendications**

1. Système d'évaluation de démarche (100), comprenant :

   un capteur de valeurs de mesure quantitatif (40, 80) qui acquiert une valeur de mesure d'un paramètre quantitatif indiquant une quantité de marche, mesurée par un dispositif de mesure (20) porté par un sujet ;
   un capteur de valeurs de mesure qualitatif qui acquiert au moins des valeurs de mesure d'une longueur de foulée, d'un angle de balancement des bras et d'une cadence en tant que paramètres qualitatifs indiquant une forme de marche, mesurés par un dispositif de mesure (20) porté par le sujet ;
   une unité de détermination de résultat quantitatif qui détermine un résultat d'évaluation concernant une quantité de marche, sur la base d'une valeur de mesure du paramètre quantitatif ;
   une unité de détermination de résultat qualitatif (96) qui détermine un résultat d'évaluation concernant une forme de marche, sur la base d'un modèle d'évaluation prédéterminé qui reflète le degré d'approximation d'une tendance de forme avec laquelle un effet d'exercice de marche est susceptible d'être obtenu, et des valeurs de mesure des paramètres qualitatifs ; et
   une unité de sortie qui délivre des informations de résultat de marche comprenant le résultat d'évaluation concernant la quantité de marche et le résultat d'évaluation concernant la forme de marche,

dans lequel l'unité de détermination de résultat qualitatif (96) détermine le résultat d'évaluation concernant la forme de marche en utilisant, comme modèle d'évaluation prédéterminé, une formule d'évaluation pour obtenir la somme d'une valeur obtenue en ajoutant un poids au rapport du rapport de la hauteur de foulée du sujet à un rapport de hauteur de foulée moyen d'un niveau attribué correspondant, et une valeur du rapport de la cadence du sujet à une cadence moyenne du niveau attribué correspondant, et une formule d'évaluation qui est définie de telle sorte qu'un poids est ajouté au moins à une valeur de mesure de l'angle de balancement des bras.

2. Système d'évaluation de démarche (100) selon la revendication 1, dans lequel l'unité de détermination de résultat qualitatif (96) détermine le résultat d'évaluation concernant la forme de marche en utilisant, comme modèle d'évaluation prédéterminé, une formule d'évaluation qui est définie de telle sorte qu'un poids est ajouté au moins à une valeur de mesure de la longueur de foulée.

3. Système d'évaluation de démarche (100) selon la revendication 1 ou 2, dans lequel la valeur de mesure de la longueur de foulée est un rapport d'une longueur de foulée moyenne à la taille du sujet mesurée.

4. Système d'évaluation de démarche (100) selon l'une des revendications 1 à 3, dans lequel

   le capteur de valeurs de mesure qualitatif acquiert en outre une valeur de mesure du mouvement de bassin en tant que paramètres qualitatifs, et
   l'unité de détermination de résultat qualitatif (96) détermine le résultat d'évaluation concernant la forme de marche en utilisant, comme modèle d'évaluation prédéterminé, une formule d'évaluation qui est définie de telle sorte qu'un poids est ajouté au moins à une valeur de mesure du mouvement de bassin.

5. Système d'évaluation de démarche (100) selon l'une des revendications 1 à 4, dans lequel l'unité de sortie délivre les informations de résultat de marche comprenant des informations indiquant si au moins l'un parmi le résultat d'évaluation concernant la quantité de marche ou le résultat d'évaluation concernant la forme de marche a satisfait à une condition d'atteinte cible prédéterminée.

**6.** Système d'évaluation de démarche (100) selon l'une des revendications 1 à 5, dans lequel l'unité de sortie délivre les informations de résultat de marche comprenant des informations concernant un effet de l'exercice sur chaque partie du corps en fonction du résultat d'évaluation concernant la quantité de marche et le résultat d'évaluation concernant la forme de marche.

**7.** Système d'évaluation de démarche (100), comprenant :

un capteur de valeurs de mesure quantitatif (40, 80) qui acquiert une valeur de mesure d'un paramètre quantitatif indiquant une quantité de marche, mesurée par un dispositif de mesure (20) porté par un sujet ;
un capteur de valeurs de mesure qualitatif qui acquiert au moins des valeurs de mesure du mouvement de bassin, d'un angle de balancement des bras et d'une cadence en tant que paramètres qualitatifs indiquant une forme de marche, mesurés par un dispositif de mesure (20) porté par le sujet ;
une unité de détermination de résultat quantitatif qui détermine un résultat d'évaluation concernant une quantité de marche, sur la base d'une valeur de mesure du paramètre quantitatif ;
une unité de détermination de résultat qualitatif (96) qui détermine un résultat d'évaluation concernant une forme de marche, sur la base d'un modèle d'évaluation prédéterminé qui reflète le degré d'approximation d'une tendance de forme avec laquelle un effet d'exercice de marche est susceptible d'être obtenu, et des valeurs de mesure des paramètres qualitatifs ; et
une unité de sortie qui délivre des informations de résultat de marche comprenant le résultat d'évaluation concernant la quantité de marche et le résultat d'évaluation concernant la forme de marche,
dans lequel l'unité de détermination de résultat qualitatif (96) détermine le résultat d'évaluation concernant la forme de marche en utilisant, comme modèle d'évaluation prédéterminé, une formule d'évaluation pour obtenir la somme d'une valeur obtenue en ajoutant un poids au rapport du rapport de la hauteur de foulée du sujet à un rapport de hauteur de foulée moyen d'un niveau attribué correspondant, et une valeur du rapport de la cadence du sujet à une cadence moyenne du niveau attribué correspondant, et une formule d'évaluation qui est définie de telle sorte qu'un poids est ajouté au moins à une valeur de mesure de l'angle de balancement des bras.

**8.** Procédé d'évaluation de démarche, comprenant :

l'acquisition d'une valeur de mesure d'un paramètre quantitatif indiquant une quantité de marche, mesurée par un dispositif de mesure (20) porté par un sujet ;
l'acquisition d'au moins des valeurs de mesure d'une longueur de foulée, d'un angle de balancement des bras et d'une cadence en tant que paramètres qualitatifs indiquant une forme de marche, mesurés par un dispositif de mesure (20) porté par le sujet ;
la détermination d'un résultat d'évaluation concernant une quantité de marche, sur la base d'une valeur de mesure du paramètre quantitatif ;
la détermination d'un résultat d'évaluation concernant une forme de marche, sur la base d'un modèle d'évaluation prédéterminé qui reflète le degré d'approximation d'une tendance de forme avec laquelle un effet d'exercice de marche est susceptible d'être obtenu, et des valeurs de mesure des paramètres qualitatifs ; et
la sortie des informations de résultat de marche comprenant le résultat d'évaluation concernant la quantité de marche et le résultat d'évaluation concernant la forme de marche,
dans lequel, en déterminant le résultat d'évaluation concernant une forme de marche, la détermination du résultat d'évaluation concernant la forme de marche en utilisant, comme modèle d'évaluation prédéterminé, une formule d'évaluation pour obtenir la somme d'une valeur obtenue en ajoutant un poids au rapport du rapport de la hauteur de foulée du sujet à un rapport de hauteur de foulée moyen d'un niveau attribué correspondant, et une valeur du rapport de la cadence du sujet à une cadence moyenne du niveau attribué correspondant, et une formule d'évaluation qui est définie de telle sorte qu'un poids est ajouté au moins à une valeur de mesure de l'angle de balancement des bras.

FIG. 1

FIG. 2

FIG. 3

STRIDE LENGTH [cm]

150   120   90   60   30   0

WALKING TIME [min]

0   10   20   30   40

110

112

114

MUSCLE ACTIVITY LEVEL ESTIMATE
WALKING SPEED [km/h]

10   9   8   7   6   ⑤   4   3   2   1   0

FIG. 4

122

120

123    125

124

YOU    GENERATION'S AVERAGE

123

POSITIVE    NEGATIVE

127

ARM SWING ANGLE

52°

126

128

FIG. 5

MEASUREMENT RESULTS

HIP ROTATION ANGLE
20.5°

HIP SWAY ON LEFT
AND RIGHT SIDES
17.3°

DIFFERENCE BETWEEN LEFT
AND RIGHT CADENCES
105 MILLISECONDS

FIG. 6

FIG. 7

EXERCISE EFFECT ON EACH BODY PART

**EP 4 324 391 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022051173 A
- JP 2020174946 A
- AU 2021107366 A4